# EUROPEAN PATENT APPLICATION

(11) **EP 4 070 854 A1**
(43) Date of publication of application: **12.10.2022**
(21) Application number: 20895983.3
(22) Date of filing: 03.12.2020
(51) Int. Cl.: A61P 1/00, A61P 29/00, A61P 43/00, A61K 36/22, A23K 20/158, A23K 50/10, A23K 50/30, A23K 50/75, A61K 31/05, A61K 31/192

(54) **ANTI-INFLAMMATORY AGENT FOR INTESTINAL CELL**

(30) Priority: 03.12.2019 JP 2019218881
(71) Applicant: Idemitsu Kosan Co., Ltd, Tokyo 100-8321 (JP)
(72) Inventor: HIYAMUTA, Shuichi, Tsukuba-shi, Ibaraki 300-2646 (JP); HIKITA, Chie, Tsukuba-shi, Ibaraki 300-2646 (JP); KITAZAWA, Haruki, Sendai-shi, Miyagi 980-8577 (JP)
(74) Representative: Vossius & Partner Patentanwälte Rechtsanwälte mbB
(86) International application number: PCT/JP2020/045013
(87) International publication number: WO 2021/112171

(57) **Abstract**

An agent for suppressing inflammation in the intestinal cell of a non-human animal comprises cashew nut shell liquid, heated cashew nut shell liquid, anacardic acid, cardanol, and/or cardol.

## Description

### TECHNICAL FIELD

The present invention relates to: an agent and feed for suppressing inflammation of the intestinal cells of a ruminant, or a nonhuman animal such as a pig or poultry; and a method for suppressing inflammation of the intestinal cells of the nonhuman animal using the agent and the feed.

### BACKGROUND ART

The health condition maintenance and disease control of domestic animals are important factors for improving the productivity of the domestic animals such as ruminants, pigs, and poultry.

LPS (lipopolysaccharide) and the like which are the decomposition products of pathogenic bacteria cause intestinal inflammation in the intestines of the domestic animals, resulting in various gastrointestinal diseases including diarrhea and colitis, decreases in resistance and immunity due to nutritional deprivation, and susceptibility to diseases, caused by the decreases, which have been greatly problematic in the field of animal industry.

In contrast, live microbial agents such as *Bacillus* bacteria and bifidobacteria have also been used in the animal industry for the purpose of intestinal regulation effects. However, there have not been any natural product formulations confirmed to have the effects of suppressing the secretory of inflammatory cytokines and repairing the intestinal tight junction (TJ).

Known effects of cashew nut shell liquid on domestic animals include the effect of improving rumen fermentation (Patent Document 1), the effect of preventing tympanites (Patent Document 2), the effect of preventing coccidiosis (Patent Document 3), and the effect of preventing acidosis (Patent Document 4). However, there is no reports so far with regards to the effects of cashew nut shell oil to suppress the intestinal inflammation of domestic animals.

### PRIOR ART DOCUMENTS

### Patent Documents

Patent Document 1: WO 2008/149992
Patent Document 2: WO 2008/149994
Patent Document 3: WO 2009/139468
Patent Document 4: WO 2010/053085

### SUMMARY OF THE INVENTION

### Problems to be solved by the invention

An object of the present invention is to provide a material for suppressing the intestinal inflammation of a non-human animal.

### Means to solve the problems

The inventors intensively studied to solve the above-described problems and consequently found that an action of suppressing inflammation of the intestinal cells of an animal is provided by cashew nut shell liquid (hereinafter, may be abbreviated as "CNSL" or "CNSE"). The inventors completed the present invention on the basis of such findings.

That is, the present invention is as follows.
[1] A method of suppressing inflammation in an intestinal cells of a nonhuman animal, comprising administering cashew nut shell liquid, heated cashew nut shell liquid, anacardic acid, cardanol, and/or cardol to a nonhuman animal.
[2] A method of suppressing inflammation in an intestinal cells of a nonhuman animal, comprising: mixing a feed with cashew nut shell liquid, heated cashew nut shell liquid, anacardic acid, cardanol, and/or cardol; and administering the feed to the non-human animal.
[3] A method of suppressing inflammation in an intestinal cells of a nonhuman animal, comprising: mixing the feed with 5 ppm to 1000 ppm (weight) of cashew nut shell liquid, heated cashew nut shell liquid, anacardic acid, cardanol, and/or cardol; and administering the feed to the nonhuman animal.
[4] The method of suppressing inflammation in intestinal cells according to any of [1] to [3], wherein the nonhuman animal is a mammal.
[5] The method of suppressing inflammation in intestinal cells according to any of [1] to [3], wherein the non-human animal is poultry, a pig, or a ruminant.
[6] The method of suppressing inflammation in intestinal cells according to any of [1] to [3], wherein the non-human animal is a ruminant.
[7] The method of suppressing inflammation in intestinal cells in a non-human animal according to any of [1] to [6], wherein the administration of cashew nut shell liquid, heated cashew nut shell liquid, anacardic acid, cardanol, and/or cardol results in suppression of production of an inflammatory cytokine in intestinal cells to suppress the inflammation in the intestinal cells of the non-human animal.
[8] The method according to [7], wherein the inflammatory cytokine is one or more of TNFα, IL-6, and MCP-1.
[9] An agent for suppressing intestinal inflammation of a non-human animal, comprising, as active components, cashew nut shell liquid, heated cashew nut shell liquid, anacardic acid, cardanol, and/or cardol.
[10] The agent for suppressing intestinal inflammation according to [9], wherein said agent comprises 5 ppm to 1000 ppm (weight) of cashew nut shell liquid, heated cashew nut shell liquid, anacardic acid, cardanol, and/or cardol.
[11] The agent for suppressing intestinal inflammation according to [9] or [10], wherein anacardic acid, cardanol, and/or cardol are active components.
[12] An agent for suppressing production of an inflammatory cytokine in intestinal cells of a nonhuman animal, comprising, as active components, cashew nut shell liquid, heated cashew nut shell liquid, anacardic acid, cardanol, and/or cardol.
[13] The agent for suppressing intestinal inflammation according to [12], wherein said agent comprises 5 ppm to 1000 ppm (weight) of cashew nut shell liquid, heated cashew nut shell liquid, anacardic acid, cardanol, and/or cardol.
[14] The agent for suppressing intestinal inflammation according to [12] or [13], wherein anacardic acid, cardanol, and/or cardol is/are active components.
[15] Use of cashew nut shell liquid, heated cashew nut shell liquid, anacardic acid, cardanol, and/or cardol in manufacturing an agent for suppressing production of an inflammatory cytokine in intestinal cells of a non-human animal.

### EFFECTS OF THE INVENTION

In accordance with the present invention, secretion of an inflammatory cytokine secreted from intestinal cells can be suppressed by administering an agent or feed comprising cashew nut shell liquid, heated cashew nut shell liquid, anacardic acid, cardanol, and/or cardol to a non-human animal such as a ruminant, a pig, or poultry. As a result, the onset and symptomatic worsening of inflammation in the intestinal cells can be suppressed. As a result, the health condition of the non-human animal such as a domestic animal can be maintained to improve productivity. These substances are from natural substances and thus is considered to suppress side effects with low appearance of resistant bacteria. These substances have an advantage of acting in a low dose and also having high safety.

### BRIEF DESCRIPTION OF DRAWINGS

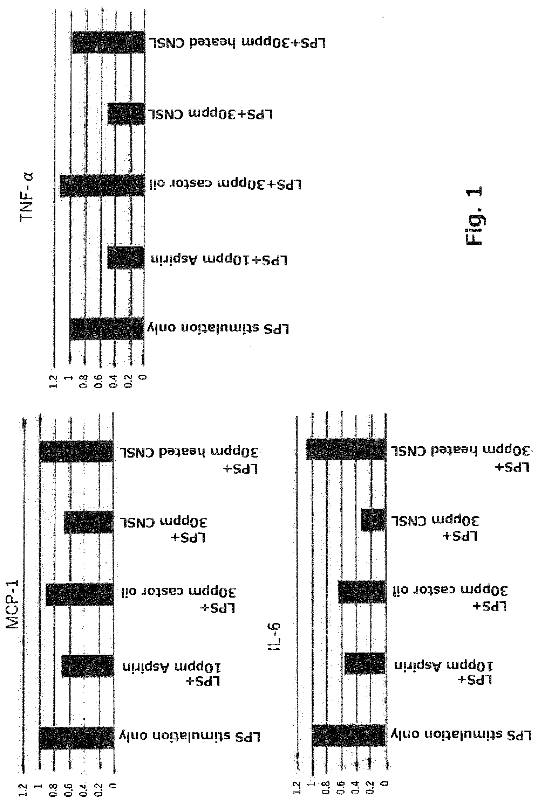

[Figure 1] Figure 1 is a graph indicating the results (RT-PCR) of the analysis of the expression of an inflammatory cytokine gene in the case of adding various substances to porcine intestinal epithelial cells. Their effects on LPS-stimulated increase in the expression of the inflammatory cytokine gene were examined. The ordinate indicates a relative expression level on the assumption that an expression level at LPS stimulation only is set at 1.

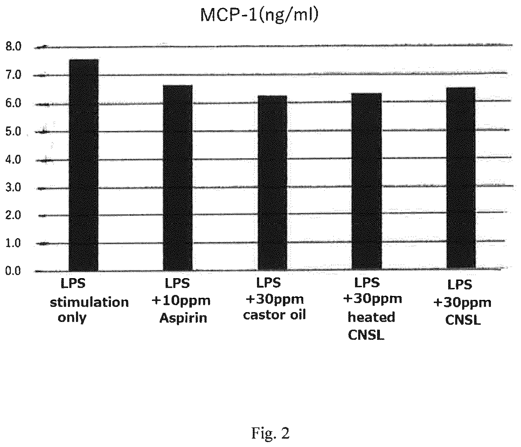

[Figure 2] Figure 2 is a diagram indicating the results of the quantification of the secretion amount of MCP-1 in the case of adding various substances to porcine intestinal epithelial cells. Their effects on LPS-stimulated increase in the secretion of MCP-1 were examined.

### DETAILED DESCRIPTION OF THE INVENTION

The agent for suppressing inflammation in intestinal cells of the present invention comprises, as active components, cashew nut shell liquid (CNSL), heated cashew nut shell liquid, anacardic acid, cardanol, and/or cardol.

Examples of suppression of inflammation in intestinal cells include suppression of an inflammatory symptom in intestinal cells or tissues, and suppression of production of an inflammatory cytokine in intestinal cells. The suppression of the production of the inflammatory cytokine includes not only suppression of secretion of an inflammatory cytokine but also suppression of expression of an inflammatory cytokine gene. Examples of the inflammatory cytokine include MCP-1 (monocyte chemoattractant protein-1), IL-6 (interleukine-6), and TNF-α (tumor necrosis factor-α).

An intestinal epithelial cell is preferred as the intestinal cell.

The agent for suppressing of inflammation in intestinal cells of the present invention can also be used as an agent for suppressing production of an inflammatory cytokine in intestinal cells. The agent for suppressing production of an inflammatory cytokine preferably results in decreases in the production amount of the inflammatory cytokine to 90% or less, preferably 80% or less, and more preferably 50% or less, as compared with production amount in a control (without addition of CNSL, heated cashew nut shell liquid, anacardic acid, cardanol, and/or cardol).

Suppression of production of an inflammatory cytokine, evoked by an inflammatory stimulus, is preferred as the suppression of production of an inflammatory cytokine. Examples of the inflammatory stimulus include a stimulus derived from bacteria, such as LPS (lipopolysaccharide). As a result, inflammation caused by a bacterial infection or the like can be efficiently suppressed.

The agent for suppressing of inflammation in intestinal cells and the agent for suppressing production of an inflammatory cytokine in intestinal cells, of the present invention, can be preferably used in prevention or treatment of a disease caused by inflammation in intestinal cells. Examples of such diseases include bacterial infections and inflammatory bowel diseases.

Cashew nut shell liquid is an oily liquid contained in the nutshell of the cashew nut tree (*Anacardium occidentale* L.). Cashew nut shell liquid contains, as its components, anacardic acid, cardanol, and cardol. Anacardic acid is converted into cardanol by heat treatment. Cashew nut shell liquid containing only cardanol and cardol due to heat treatment (heated cashew nut shell liquid) may also be used.

Cashew nut shell liquid (unheated) extracted by pressing cashew nut shells comprises 55 to 80% by mass of anacardic acid, 5 to 20% by mass of cardanol and 5 to 30% by mass of cardol, as described in J. Agric. Food Chem. 2001, 49, 2548-2551.

Heated cashew nut shell liquid obtained by heat treatment of unheated cashew nut shell liquid (for example, at 70°C or more, preferably 130°C or more), causing anacardic acid, which is a main component of unheated cashew nut shell liquid, to be decarboxylated and converted into cardanol, comprises 0 to 10% by mass of anacardic acid, 55 to 80% by mass of cardanol, and 5 to 30% by mass of cardol.

Cashew nut shell liquid can be obtained as a vegetable oil extracted by pressing cashew nut shells. Cashew nut shell liquid can also be obtained by dry distillation or solvent extraction of cashew nut shells. For example, cashew nut shell liquid can also be obtained by a method as described in Japanese Unexamined Patent Publication No. H08-231410. A commercial product of cashew nut shell liquid may be used.

The agent for suppressing inflammation in an intestinal cell of the present invention, may comprise anacardic acid, cardanol, and cardol, instead of cashew nut shell liquid.

Examples of anacardic acid include naturally-occurring anacardic acid, synthetic anacardic acid, and derivatives thereof. Moreover, a commercial product of anacardic acid may also be used. Anacardic acid can be obtained as described in Japanese Unexamined Patent Publication No. H08-231410 by extracting cashew nut oil from cashew nut shells treated with an organic solvent, subjecting the obtained cashew nut oil to, for example, silica gel column chromatography, and applying a mixed solvent of n-hexane, ethyl acetate and acetic acid with a varying ratio to the chromatography for the elution of the obtained cashew nut oil (see Japanese Unexamined Patent Publication No. H03-240721, Japanese Unexamined Patent Publication No. H03-240716, etc.).

Examples of cardanol include naturally-occurring cardanol, synthetic cardanol, and derivatives thereof. Moreover, cardanol to be used in the present invention can be obtained by decarboxylation of anacardic acid, a main component of cashew nut shell liquid.

Examples of cardol include naturally-occurring cardol, synthetic cardol, and derivatives thereof. Moreover, cardol to be used in the present invention can be obtained from purification of cashew nut shell liquid.

The content of cashew nut shell liquid, heated cashew nut shell liquid, anacardic acid, cardanol, and/or cardol in the agent for suppressing inflammation in intestinal cells of the present invention, is preferably 0.5 to 50,000 weight ppm, more preferably 1 to 10,000 ppm, furthermore preferably 5 to 1,000 ppm based on the total amount of the agent.

As the agent for suppressing inflammation in intestinal cells of the present invention, an undiluted solution of cashew nut shell liquid, heated cashew nut shell liquid, anacardic acid, cardanol, and/or cardol may be directly administered orally.

The agent for suppressing inflammation in intestinal cells of the present invention, may comprise components such as an excipient which can be used in a feed or pharmaceutical product, such as, for example, lactose, sucrose, D-mannitol, α-starch, starch, corn starch, crystalline cellulose, bentonite, silica gel, or light anhydrous silicic acid, in addition to cashew nut shell liquid, heated cashew nut shell liquid, anacardic acid, cardanol, and/or cardol.

The agent for suppressing inflammation in intestinal cells of the present invention, may further comprise an optional component such as a component effective in promotion of the growth of a ruminant, a nutritional component, a component enhancing storage stability, or a coating component, in addition to cashew nut shell liquid, heated cashew nut shell liquid, anacardic acid, cardanol, and/or cardol. Examples of such optional components include raw materials of feeds, such as bran, alfalfa, and timothy, feed additives, food source materials, food additives, pharmaceutical product materials, and other supplement components used in supplements for animals (hereinafter referred to as "supplements"). Examples thereof include: living bacterial agents such as bacteria belonging to the genus *Enterococcus*, bacteria belonging to the genus *Bacillus*, and *Lactobacillus bifidus*; enzymes such as amylase and lipase; L-ascorbic acid, choline chloride, inositol, and vitamins such as folic acid; minerals such as potassium chloride, ferric citrate, magnesium oxide, and phosphates; amino acids such as DL-alanine, DL-methionine, and L-lysine; organic acids such as fumaric acid, butyric acid, lactic acid, and acetic acid, and salts thereof; antioxidants such as ethoxyquin, dibutyl hydroxytoluene, butylated hydroxyanisole, ferulic acid, vitamin C, and vitamin E; fungicides such as calcium propionate; binding agents such as carboxymethyl cellulose (CMC), sodium caseinate, and sodium polyacrylate; emulsifiers such as lecithin, glycerin fatty acid ester, and sorbitan fatty acid ester; coloring agents such as astaxanthin and canthaxanthin; and flavoring agents such as various esters, ethers, and ketones. Kinds of supplements and the components other than cashew nut shell liquid, heated cashew nut shell liquid, anacardic acid, cardanol, and/or cardol are not limited.

The agent for suppressing inflammation in intestinal cells of the present invention, may comprise an oil absorption agent such as magnesium oxide, stearate, talc, zeolite, diatom earth, or silica, and the oil absorption agent is preferably granulous. The oil absorption agent is preferably an oil absorption agent that can adsorb 50 to 300 g of oil per 100 g of the oil absorption agent. When the oil absorption agent has a particle diameter of more than 300 µm, the particles of the oil absorption agent are coarsened and separated. Therefore, the oil absorption agent preferably has a particle diameter of 2 to 300 µm.

In one embodiment of the agent for suppressing inflammation in intestinal cells of the present invention, a preferred mass ratio between the oil absorption agent and cashew nut shell liquid (CNSL), heated cashew nut shell liquid, anacardic acid, cardanol, and/or cardol is 100:20 to 100:180. A preferred mass ratio between the oil absorption agent and ground cashew nut shell product is 15:100 to 60:100.

The dosage form for the agent for suppressing inflammation in intestinal cells of the present invention, is not particularly limited, but examples thereof include any forms such as liquid medicine, powder, solid, tablet, capsule, emulsion, pellet, tablet, and coating. The dosage form is preferably liquid medicine, powder, capsule, pellet, or tablet.

Cashew nut shell liquid, heated cashew nut shell liquid, anacardic acid, cardanol, and/or cardol may be used on an as-is basis as the liquid agent. Alternatively, cashew nut shell liquid, heated cashew nut shell liquid, anacardic acid, cardanol, and/or cardol may be dissolved in a solvent such as ethanol. Further, the above-described excipient or an optional component may be added thereto. The powder, capsule, pellet, or tablet as described below may be suspended and allowed to float in a liquid.

As for the powder, the above-described excipient may be added to cashew nut shell liquid, heated cashew nut shell liquid, anacardic acid, cardanol, and/or cardol to make the powder.

As for the capsule, a capsule may be filled with cashew nut shell liquid, heated cashew nut shell liquid, anacardic acid, cardanol, and/or cardol on an as-is basis, or the above-described excipient or an optional component may be added to the capsule.

As for the pellet, the above-described excipient may be added to cashew nut shell liquid, heated cashew nut shell liquid, anacardic acid, cardanol, and/or cardol, and the resultant may be granulated to make the pellet.

As for the tablet, the above-described excipient may be added to cashew nut shell liquid, heated cashew nut shell liquid, anacardic acid, cardanol, and/or cardol, and the resultant may be granulated to make the tablet.

It is preferable to formulate the agent as the powder, the tablet, and the pellet when the agent comprises an oil absorption agent such as silica.

As described above, the agent for suppressing inflammation in intestinal cells of the present invention, can be produced by mixing cashew nut shell liquid, heated cashew nut shell liquid, anacardic acid, cardanol, and/or cardol with an excipient or an optional component, as necessary, for formulation. In addition, depending of the form of the agent, a product obtained by pulverizing/crushing the cashew nut shell, or cashew nut shell without being processed may be mixed directly with any other optional components to produce the agent for suppressing inflammation in intestinal cells of the present invention. Further, ground or crushed product of cashew nut shell or cashew nut shell itself may be used as the agent for suppressing inflammation in intestinal cells of the present invention without mixing with other optional components.

The feed of the present invention comprises cashew nut shell liquid, heated cashew nut shell liquid, anacardic acid, cardanol, and/or cardol.

The content of cashew nut shell liquid, heated cashew nut shell liquid, anacardic acid, cardanol, and/or the cardol in the feed of the present invention is preferably 0.5 to 50,000 ppm by weight, more preferably 1 to 10,000 ppm by weight, still more preferably 5 to 100 ppm by weight based on the total amount per dry matter mass of the feed.

The feed of the present invention can be produced by adding cashew nut shell liquid, heated cashew nut shell liquid, anacardic acid, cardanol, and/or cardol, or the agent for suppressing inflammation in an intestinal cell of the present invention, comprising cashew nut shell liquid, heated cashew nut shell liquid, anacardic acid, cardanol, and/or cardol to a feed component, and by mixing the resultant.

In such a case, when the agent which is powdery and solid is used, the agent may be allowed to be in a liquid or a gel form using a liquid carrier to facilitate the mixture. In this case, a flowable liquid such as water, vegetable oil, liquid animal oil, mineral oil, synthetic oil, or a water-soluble polymer compound can be used as the liquid carrier. Moreover, water-soluble polysaccharides such as alginic acid, sodium alginate, xanthan gum, carboxymethyl cellulose, α-starch, sodium caseinate, gum arabic, guar gum, and tamarind seed polysaccharides are also preferably combined to keep the homogeneity of cashew nut shell liquid, heated cashew nut shell liquid, anacardic acid, cardanol, and/or cardol in the feed.

For the feed of the present invention, the kinds, combination rates, and the like of feed components combined with the agent of the present invention are not particularly limited. The feed may be a feed conventionally given to each animal. The feed can be prepared using, for example, corn grains, corn flours, milo, bran, soybean cake, oats, wheat short, wheat coarse powders, alfalfa, timothy, clovers, defatted rice bran, northern ocean meal, coast meal, yeast, molasses, meat pieces, bone meal, calcium carbonate, calcium diphosphate, yellow grease, vitamins, minerals, and/or the like.

The kinds of non-human animals to be administered with the agent for suppressing inflammation in intestinal cells or the feed of the present invention are not particularly limited, but are preferably non-human mammalian animals and poultry, and examples thereof include ruminants, pigs, and poultry. Examples of the ruminants include cattle, buffalos, goats, sheep, and yaks. Examples of the kinds of the cattle include, but are not limited to, female Holstein, Jersey, Japanese black, Japanese Shorthorn, and Aberdeen Angus. Examples of the poultry include chickens, quails, turkeys, ducks, gooses, ostriches, emus, pheasants, and guinea fowl.

The amount of the agent for suppressing inflammation in intestinal cells or the feed to be given may be appropriately adjusted depending on the kind, body weight, age, sex, and health condition of the animal, feed components, and the like. In this case, the amounts of cashew nut shell liquid, heated cashew nut shell liquid, anacardic acid, cardanol, and/or cardol contained in the feed are, for example, 0.01 to 500 g/animal/day, preferably 0.1 to 200 g/animal/day, more preferably 1 to 50 g/animal/day, and furthermore preferably 0.5 to 5 g/animal/day.

### EXAMPLES

The present invention will be specifically described below with reference to Examples. However, aspects of the present invention are not limited to the following Examples.

To evaluate the effect of endotoxin stimulated inflammatory reaction in the intestine, CNSL, castor oil, or heated CNSL was added to porcine intestinal epithelial cells, the cells were cultured, and cytokine genes and cytokine proteins were quantified.

Testing method
Evaluation of Cell Culture, LPS Stimulation, and Anti-inflammatory Substance

### 1. Seeding of PIE Cells

PIE (porcine intestinal epithelial) cells were seeded in a 12-well plate (manufactured by COSTAR) at 3.0 × 10⁴ cells/well, and cultured in a CO₂ incubator. (Day 0)

### 2. Added Samples

- Medium (DMEM Dulbecco's modified MEM medium) as NC (negative control)
   PC1 (positive control 1)·Acetylsalicylic acid (aspirin)
   Aspirin (manufactured by BAYER) is ground in a mortar, and PBS (phosphate buffered saline) solution containing 10 mg/mL of aspirin is then produced and sterilized through a filter of 0.45 µm (manufactured by ADVANTEC TOYO). The sterilized PBS is used to prepare the diluted solution of Aspirin (400 µg/mL or 200 µg/mL) and the diluted solution is added to the medium at the amount of 1/20 volume (Final concentration of 20 ppm or 10 ppm).
   PC2 (positive control 2) Castor oil
- CNSE (cashew nut shell liquid)
- CNSL (heated cashew nut shell liquid)
   10 mg/mL castor oil, or ethanol solution (stock solution) containing 50 mg/mL (CNSE or heated CNSL) is diluted with PBS to produce 5 mg/mL, 2. 5 mg/mL or 1.25 mg/mL, 0.6 mg/mL or 0.3 mg/mL of solution, which is then sterilized through a filter (0.45 µm).

### 3. Medium Replacement

20 µL of the above-described samples is added to 1 mL of DMEM to prepare a medium for replacement.

The medium in the 12-well plate is aspirated and replaced with the medium for replacement (at 9:00 a.m. on Day 3)

### LPS Stimulation

An LPS (lipopolysaccharide) solution is added to the medium to achieve a final concentration of 1 µg/mL (at 9:00 p.m. on Day 4).

### Harvest I

A culture supernatant is collected, the wells are then washed with the sterilized PBS, and adherent cells were solubilized with TRIZOL, and harvested (at 9:00 a.m. on Day 5).

### Harvest II

A culture supernatant is collected, the wells are then washed with the sterilized PBS, and adherent cells are solubilized with TRIZOL, and harvested (at 9:00 a.m. on Day 7).

### RT-PCR (using the sample of Harvest I)

The sample in which TRIZOL (manufactured by INVITROGEN) is dissolved is partially purified by phenol-chloroform extraction and ethanol precipitation, and then dissolved in 10 µL of RNase-free water, and the O.D. (absorbance) of the resultant is measured with NanoDrop (manufactured by Thermo Fisher Scientific K.K.).

Real time PCR was performed in conformity with the manual of Prime Script RT reagent Kit with g DNA Eraser of TAKARABIO.

### ELISA (using the sample of Harvest II)

MCP-1 was quantified according to the protocol of Ray Bio porcine C-C motif chemokine 2 (CCL2)/Monocyte chemoattractant Protein 1 (MCP1) ELISA Kit. The culture supernatant was diluted twice for the quantification. The standard was 2800pg/ml to 28.67pg/ml.

### Results

The results of RT-PCR are set forth in Figure 1. The results reveal that LPS-induced increases in the expression of MCP-1, IL-6, and TNF-α were suppressed by CNSL. Also, as shown in Figure 2, both CNSL and heated CNSL suppressed the LPS-induced increase of MCP-1 secretion.

### Discussion

As described above, it was suggested that CNSL and heated CNSL suppress the action of the inflammation mediators, stimulated by endotoxin (LPS: lipopolysaccharide), in PIE cells (porcine intestinal epithelial cells).

## Claims

1. A method of suppressing inflammation in intestinal cells of a non-human animal, comprising administering cashew nut shell liquid, heated cashew nut shell liquid, anacardic acid, cardanol, and/or cardol to a non-human animal.

2. A method of suppressing inflammation in intestinal cells of a non-human animal, comprising: mixing a feed with cashew nut shell liquid, heated cashew nut shell liquid, anacardic acid, cardanol, and/or cardol; and administering the feed to a non-human animal,

3. A method of suppressing inflammation in intestinal cells of a non-human animal, comprising: mixing a feed with 5 ppm to 1000 ppm (weight) of cashew nut shell liquid, heated cashew nut shell liquid, anacardic acid, cardanol, and/or cardol; and administering the feed to the non-human animal.

4. The method of suppressing inflammation in intestinal cells according to any one of claims 1 to 3, wherein the non-human animal is a mammal.

5. The method of suppressing inflammation in intestinal cells according to any one of claims 1 to 3, wherein the non-human animal is poultry, a pig, or a ruminant.

6. The method of suppressing inflammation in intestinal cells according to any one of claims 1 to 3, wherein the non-human animal is a ruminant.

7. The method of suppressing inflammation in intestinal cells in a non-human animal according to any one of claims 1 to 6, wherein the administration of cashew nut shell liquid, heated cashew nut shell liquid, anacardic acid, cardanol, and/or cardol results in suppression of production of an inflammatory cytokine in intestinal cells to suppress inflammation in intestinal cells of the non-human animal.

8. The method according to claim 7, wherein the inflammatory cytokine is one or more of TNFα, IL-6, and MCP-1.

9. An agent for suppressing intestinal inflammation of a non-human animal, comprising, as active components, cashew nut shell liquid, heated cashew nut shell liquid, anacardic acid, cardanol, and/or cardol.

10. The agent for suppressing intestinal inflammation according to claim 9, wherein said agent comprises 5 ppm to 1000 ppm (weight) of cashew nut shell liquid, heated cashew nut shell liquid, anacardic acid, cardanol, and/or cardol.

11. The agent for suppressing intestinal inflammation according to claim 9 or 10, wherein anacardic acid, cardanol, and/or cardol is/are active components.

12. An agent for suppressing production of an inflammatory cytokine in intestinal cells of a non-human animal, comprising, as active components, cashew nut shell liquid, heated cashew nut shell liquid, anacardic acid, cardanol, and/or cardol.

13. The agent for suppressing production of an inflammatory cytokine according to claim 12, wherein said agent comprises 5 ppm to 1000 ppm (weight) of cashew nut shell liquid, heated cashew nut shell liquid, anacardic acid, cardanol, and/or cardol.

14. The agent for suppressing production of an inflammatory cytokine according to claim 12 or 13, wherein anacardic acid, cardanol, and/or cardol is/are active components.

15. Use of cashew nut shell liquid, heated cashew nut shell liquid, anacardic acid, cardanol, and/or cardol in manufacturing an agent for suppressing production of an
inflammatory cytokine in intestinal cells of a non-human animal.
